# EUROPEAN PATENT APPLICATION

(11) **EP 4 847 483 A1**
(43) Date of publication of application: **02.12.2026**
(21) Application number: 25808918.4
(22) Date of filing: 09.04.2025
(51) Int. Cl.: C07C 65/05, C07C 229/38, C07C 227/18, C07C 51/41, A61K 31/205, A61K 31/60, A61P 17/00, A61K 47/55, A61K 8/44, A61K 8/368, A61Q 19/00, A61Q 19/02

(54) **SUPRAMOLECULAR 4-METHOXYSALICYLIC ACID TRANEXAMIC ACID IONIC SALT, PREPARATION METHOD THEREFOR, AND USE THEREOF**

(30) Priority: 06.05.2024 CN 202410549041
(71) Applicant: Shenzhen Shinehigh Innovation Co., Ltd., Shenzhen, Guangdong 518000 (CN); GD Shinesky Medical Supplies Health Technology Co., Ltd., Shaoguan, Guangdong 512000 (CN)
(72) Inventor: WANG, Zhenyuan, Shenzhen, Guangdong 518000 (CN); ZHANG, Jiaheng, Shenzhen, Guangdong 518000 (CN); WANG, Mi, Shenzhen, Guangdong 518000 (CN); HAN, Zhixuan, Shenzhen, Guangdong 518000 (CN); YAO, Yuanzhi, Shenzhen, Guangdong 518000 (CN); TAN, Yuanzhen, Shenzhen, Guangdong 518000 (CN); CHEN, Xian, Shenzhen, Guangdong 518000 (CN); ZHANG, Jichuan, Shenzhen, Guangdong 518000 (CN)
(74) Representative: ABG Intellectual Property Law, S.L.
(86) International application number: PCT/CN2025/088124
(87) International publication number: WO 2025/232444

(57) **Abstract**

The present disclosure relates to the technical field of compounds for medicine and cosmetics, and provides a supramolecular 4-methoxysalicylic acid tranexamic acid ionic salt, a preparation method therefor, and a use thereof. The supramolecular 4-methoxysalicylic acid tranexamic acid ionic salt provided by the present disclosure can not only effectively solve the problem of poor transdermal permeability of potassium 4-methoxysalicylate, but also effectively reduce toxic and side effects and irritation of potassium 4-methoxysalicylate and tranexamic acid when used alone, thereby expanding the application range of the supramolecular 4-methoxysalicylic acid tranexamic acid ionic salt to a great extent. In addition, the 4-methoxysalicylic acid structure and the tranexamic acid structure in the supramolecular 4-methoxysalicylic acid tranexamic acid ionic salt have a synergistic effect, and compared with the potassium 4-methoxysalicylate used alone or the tranexamic acid used alone, the supramolecular 4-methoxysalicylic acid tranexamic acid ionic salt can effectively enhance the whitening effect, brightening effect, spot-fading effect, sallowness reduction effect, acne mark fading effect, and transdermal effect. Therefore, the supramolecular 4-methoxysalicylic acid tranexamic acid ionic salt can be used for the preparation of drugs, cosmetics, and skin care products having the effects of whitening, spot fading, acne mark fading, etc.

## Description

### CROSS-REFERENCE TO THE RELATED APPLICATIONS

The present invention claims the priority of the Chinese patent application with the application No. 202410549041X filed with the China National Intellectual Property Administration on May 06, 2024 and entitled "SUPRAMOLECULAR 4-METHOXYSALICYLIC ACID TRANEXAMIC ACID IONIC SALT, PREPARATION METHOD THEREFOR, AND USE THEREOF", the entire contents of which are incorporated in the present invention by reference.

### TECHNICAL FIELD

The present invention relates to the technical field of compounds for medicine and cosmetics, and in particular, to a supramolecular 4-methoxysalicylic acid tranexamic acid ionic salt, a preparation method therefor, and a use thereof.

### BACKGROUND

Potassium 4-methoxysalicylate (4MSK) is a medicinal whitening ingredient, which can coordinate keratin metabolism, correct abnormal epidermal hyperplasia, and promote melanin discharge, and promote the release of melanin by regulating the imbalance between the proliferation and differentiation of keratinocytes, thereby achieving the effects of smooth and whitened skin. However, the high water solubility of 4MSK makes it difficult for 4MSK to penetrate the stratum corneum (SC) and be delivered to the epidermis, resulting in a certain challenge for conventional 4MSK external preparations in penetrating the stratum corneum barrier, and the low transdermal permeability restricts the bioavailability of 4MSK to a certain extent. In addition, 4MSK has certain irritation, which also limits the application dosage thereof in products.

Tranexamic acid, also known as trans-4-(aminomethyl)cyclohexanecarboxylic acid, is a synthetic lysine analogue. Tranexamic acid not only has the efficacy of resisting secondary fibrinolysis, but also can inhibit the activation of plasminogen, thereby effectively alleviating ultraviolet radiation-induced melanin production and neovascularization. In addition, tranexamic acid can also reduce the activity of melanocyte tyrosinase by inhibiting the production of prostaglandins, thereby achieving the effect of whitening and spot fading. In the practical application of tranexamic acid, transdermal administration is the main using method of tranexamic acid products. However, external use of tranexamic acid may cause adverse reactions such as local erythema, skin irritation, dryness, and desquamation.

At present, both potassium 4-methoxysalicylate and tranexamic acid have certain toxicity and side effects and irritation, and how to reduce the toxicity and side effects and irritation thereof and exert better effects is a technical problem to be solved in the art.

In view of this, the present invention is hereby proposed.

### SUMMARY

An object of the present invention is to provide a supramolecular 4-methoxysalicylic acid tranexamic acid ionic salt, a preparation method therefor, and a use thereof, which aim to reduce the toxicity and side effects and irritation of potassium 4-methoxysalicylate and tranexamic acid, and to synergistically exert the spot-removing effect, whitening effect, brightening effect, anti-yellowing effect, acne mark fading effect, and transdermal effect of potassium 4-methoxysalicylate and tranexamic acid.

In a first aspect, the present invention provides a supramolecular 4-methoxysalicylic acid tranexamic acid ionic salt, the supramolecular 4-methoxysalicylic acid tranexamic acid ionic salt is a compound represented by the following structural formula, or the supramolecular 4-methoxysalicylic acid tranexamic acid ionic salt is a hydrate of the compound represented by the following structural formula:

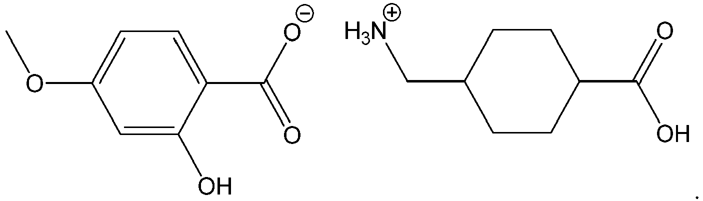

In the present invention, the 4-methoxysalicylic acid structure and the tranexamic acid structure are constructed to form a novel compound, i.e., a supramolecular 4-methoxysalicylic acid tranexamic acid ionic salt. In the supramolecular 4-methoxysalicylic acid tranexamic acid ionic salt, the 4-methoxysalicylic acid anion and the tranexamic acid cation are tightly bound through an ionic bond, which is beneficial to improving the stability of the 4-methoxysalicylic acid structure and the tranexamic acid structure in the supramolecular 4-methoxysalicylic acid tranexamic acid ionic salt in a solid phase and a liquid phase, and can effectively solve the problem of poor transdermal permeability of potassium 4-methoxysalicylate. In addition, the supramolecular 4-methoxysalicylic acid tranexamic acid ionic salt can effectively reduce the toxicity and side effects and irritation of potassium 4-methoxysalicylate and tranexamic acid when used alone, thereby expanding the application range thereof to a great extent. The 4-methoxysalicylic acid structure and the tranexamic acid structure in the supramolecular 4-methoxysalicylic acid tranexamic acid ionic salt have a synergistic effect, and compared with potassium 4-methoxysalicylate used alone or tranexamic acid used alone, the supramolecular 4-methoxysalicylic acid tranexamic acid ionic salt can effectively enhance the whitening effect, brightening effect, spot-fading effect, anti-yellowing effect, acne mark fading effect, and transdermal effect.

In combination with the first aspect, in an optional embodiment of the present invention, the supramolecular 4-methoxysalicylic acid tranexamic acid ionic salt is of an orthorhombic crystal system.

In a second aspect, the present invention provides a preparation method for the supramolecular 4-methoxysalicylic acid tranexamic acid ionic salt provided in the first aspect described above, the preparation method including: subjecting a liquid phase containing 4-methoxysalicylic acid, tranexamic acid, and an organic solvent to a heating and stirring treatment and an ultrasonic treatment in sequence, to obtain a supramolecular 4-methoxysalicylic acid tranexamic acid ionic salt solution; and subjecting the supramolecular 4-methoxysalicylic acid tranexamic acid ionic salt solution to a crystallization treatment.

In the preparation method for the supramolecular 4-methoxysalicylic acid tranexamic acid ionic salt provided by the present invention, 4-methoxysalicylic acid and tranexamic acid are used for reaction, the hydrogen atom of the 4-methoxysalicylic acid is transferred to the tranexamic acid, and proton transfer occurs, and then the 4-methoxysalicylic acid structure and the tranexamic acid structure are constructed to form a novel compound, i.e., a supramolecular 4-methoxysalicylic acid tranexamic acid ionic salt. The supramolecular 4-methoxysalicylic acid tranexamic acid ionic salt can not only effectively solve the problem of poor transdermal permeability of potassium 4-methoxysalicylate, but also effectively reduce the toxicity and side effects and irritation of potassium 4-methoxysalicylate and tranexamic acid when used alone, thereby expanding the application range thereof to a great extent. In addition, the 4-methoxysalicylic acid structure and the tranexamic acid structure in the supramolecular 4-methoxysalicylic acid tranexamic acid ionic salt have a synergistic effect, and compared with potassium 4-methoxysalicylate used alone or tranexamic acid used alone, the supramolecular 4-methoxysalicylic acid tranexamic acid ionic salt can effectively enhance the whitening effect, brightening effect, spot-fading effect, anti-yellowing effect, acne mark fading effect, and transdermal effect.

In combination with the second aspect, in an optional embodiment of the present invention, the molar ratio of the 4-methoxysalicylic acid to the tranexamic acid is (3 to 1): (1 to 3).

In the above technical solution, with the above proportion, the 4-methoxysalicylic acid and the tranexamic acid can fully react, thereby facilitating improving the yield and purity of the prepared supramolecular 4-methoxysalicylic acid tranexamic acid ionic salt.

In combination with the second aspect, in an optional embodiment of the present invention, the temperature of the heating and stirring treatment is 80 °C to 110 °C; and/or the duration of the heating and stirring treatment is 10 h to 25 h.

In the above technical solution, under the above heating and stirring treatment condition, the 4-methoxysalicylic acid and the tranexamic acid can be fully combined, which is beneficial to improving the conversion rate of the reaction between the 4-methoxysalicylic acid and the tranexamic acid, thereby facilitating improving the yield and purity of the prepared supramolecular 4-methoxysalicylic acid tranexamic acid ionic salt.

In combination with the second aspect, in an optional embodiment of the present invention, the temperature of the ultrasonic treatment is 40 °C to 60 °C; and/or the ultrasonic frequency of the ultrasonic treatment is 20 kHz to 60 kHz; and/or the ultrasonic power of the ultrasonic treatment is 280 W to 320 W; and/or the duration of the ultrasonic treatment is 4 h to 8 h.

In the above technical solution, under the above ultrasonic treatment conditions, the 4-methoxysalicylic acid and the tranexamic acid can be further fully combined and the reaction is more thorough, which is beneficial to further improving the conversion rate of the reaction between the 4-methoxysalicylic acid and the tranexamic acid, thereby facilitating further improving the yield and purity of the prepared supramolecular 4-methoxysalicylic acid tranexamic acid ionic salt.

In combination with the second aspect, in an optional embodiment of the present invention, the temperature of the crystallization treatment is ≤ 0 °C; and/or the crystallization treatment is carried out under a vacuum condition.

In the above technical solution, under the above crystallization treatment conditions, the supramolecular 4-methoxysalicylic acid tranexamic acid ionic salt solution can be fully crystallized to precipitate crystals, which is beneficial to improving the yield and purity of the prepared supramolecular 4-methoxysalicylic acid tranexamic acid ionic salt.

In combination with the second aspect, in an optional embodiment of the present invention, the preparation method for the supramolecular 4-methoxysalicylic acid tranexamic acid ionic salt solution further comprises: after the ultrasonic treatment, subjecting the system after the ultrasonic treatment to a concentration treatment; and/or the preparation method for the supramolecular 4-methoxysalicylic acid tranexamic acid ionic salt further comprises: after the crystallization treatment, sequentially filtering, washing, and drying the system after the crystallization treatment.

In combination with the second aspect, in an optional embodiment of the present invention, the organic solvent comprises at least one of methanol, ethanol, isopropanol, N-methylpyrrolidone, tetrahydrofuran, benzene, toluene, petroleum ether, n-hexane, acetone, dioxane, N,N-dimethylformamide, ethyl acetate, and acetonitrile; and/or the preparation process of the supramolecular 4-methoxysalicylic acid tranexamic acid ionic salt is carried out under an inert gas atmosphere.

Optionally, the inert gas comprises at least one of nitrogen, helium, neon, and argon.

In a third aspect, the present invention provides use of the supramolecular 4-methoxysalicylic acid tranexamic acid ionic salt provided in the first aspect described above in the preparation of a drug, a cosmetic, or a skincare product.

The supramolecular 4-methoxysalicylic acid tranexamic acid ionic salt provided by the present invention can not only effectively solve the problem of poor transdermal permeability of potassium 4-methoxysalicylate, but also effectively reduce the toxicity and side effects and irritation of potassium 4-methoxysalicylate and tranexamic acid when used alone, thereby expanding the application range thereof to a great extent. In addition, the 4-methoxysalicylic acid structure and the tranexamic acid structure in the supramolecular 4-methoxysalicylic acid tranexamic acid ionic salt have a synergistic effect, and compared with potassium 4-methoxysalicylate used alone or tranexamic acid used alone, the supramolecular 4-methoxysalicylic acid tranexamic acid ionic salt can effectively enhance the whitening effect, brightening effect, spot-fading effect, anti-yellowing effect, acne mark fading effect, and transdermal effect. Therefore, the supramolecular 4-methoxysalicylic acid tranexamic acid ionic salt can be used in the preparation of drugs, cosmetics, and skincare products having the effects of whitening, spot fading, acne mark fading, etc.

### BRIEF DESCRIPTION OF THE DRAWINGS

In order to more clearly illustrate the technical solutions of the embodiments of the present invention, the accompanying drawings required in the embodiments will be briefly introduced below. It should be understood that the following accompanying drawings only show certain embodiments of the present invention, and therefore should not be regarded as limiting the scope. For those of ordinary skill in the art, other related accompanying drawings can also be obtained based on these accompanying drawings without creative work.
FIG. 1 is a flow chart of the preparation method for the supramolecular 4-methoxysalicylic acid tranexamic acid ionic salt provided by the present invention;
FIG. 2 is a molecular structure diagram of the supramolecular 4-methoxysalicylic acid tranexamic acid ionic salt prepared in Example 1;
FIG. 3 is a ¹H NMR spectrum of the supramolecular 4-methoxysalicylic acid tranexamic acid ionic salt prepared in Example 1;
FIG. 4 is a comparison result diagram of the change rate of the facial brightness L value of the subjects in Experimental Example 6;
FIG. 5 is a comparison result diagram of the change rate of the facial erythema E value of the subjects in Experimental Example 6;
FIG. 6 is a comparison result diagram of the change rate of the facial melanin M value of the subjects in Experimental Example 6; and
FIG. 7 is a comparison result diagram of the change rate of the facial ITA° value of the subjects in Experimental Example 6.

### DETAILED DESCRIPTION OF THE EMBODIMENTS

The present invention provides a supramolecular 4-methoxysalicylic acid tranexamic acid ionic salt, wherein the supramolecular 4-methoxysalicylic acid tranexamic acid ionic salt is a compound represented by the following structural formula (Formula I), or the supramolecular 4-methoxysalicylic acid tranexamic acid ionic salt is a hydrate of the compound represented by the following structural formula (Formula I):

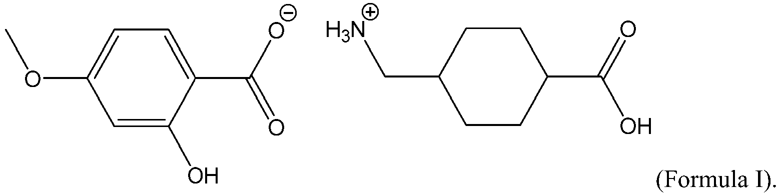

In the present invention, in Formula I, the 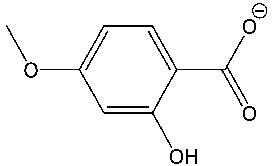 and the 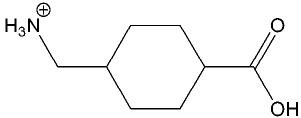 are bound through an ionic bond.

In the present invention, the chemical formula of Formula I is C₁₆H₂₃NO₆; and the chemical formula of the hydrate of Formula I is C₁₆H₂₃NO₆·nH₂O, where n is a natural number, and the specific value of n is not limited in the present invention.

In the present invention, the 4-methoxysalicylic acid structure and the tranexamic acid structure are constructed to form a novel compound, i.e., a supramolecular 4-methoxysalicylic acid tranexamic acid ionic salt. In the supramolecular 4-methoxysalicylic acid tranexamic acid ionic salt, the 4-methoxysalicylic acid anion and the tranexamic acid cation are tightly bound through an ionic bond, which is beneficial to improving the stability of the 4-methoxysalicylic acid structure and the tranexamic acid structure in the supramolecular 4-methoxysalicylic acid tranexamic acid ionic salt in a solid phase and a liquid phase, and therefore, the supramolecular 4-methoxysalicylic acid tranexamic acid ionic salt has strong chemical stability.

The supramolecular 4-methoxysalicylic acid tranexamic acid ionic salt provided by the present invention can not only effectively solve the problem of poor transdermal permeability of potassium 4-methoxysalicylate, but also effectively reduce the toxicity and side effects and irritation of potassium 4-methoxysalicylate and tranexamic acid when used alone, has good cellular and biological compatibility, has high solubility and low irritation, and can reduce irritation and discomfort to the human body, thereby expanding the application range thereof to a great extent. In addition, the 4-methoxysalicylic acid structure and the tranexamic acid structure in the supramolecular 4-methoxysalicylic acid tranexamic acid ionic salt have a synergistic effect, and compared with potassium 4-methoxysalicylate used alone or tranexamic acid used alone, the supramolecular 4-methoxysalicylic acid tranexamic acid ionic salt can improve the bioavailability of potassium 4-methoxysalicylate and tranexamic acid, has high permeability, can effectively reduce melanin production, and can effectively enhance the whitening effect, brightening effect, spot-fading effect, anti-yellowing effect, acne mark fading effect, and transdermal effect.

In the present invention, the supramolecular 4-methoxysalicylic acid tranexamic acid ionic salt is a hydrate of Formula I, and the chemical formula of the supramolecular 4-methoxysalicylic acid tranexamic acid ionic salt is C₁₆H₂₃NO₆·H₂O (i.e., C₁₆H₂₅NO₇).

In the present invention, the supramolecular 4-methoxysalicylic acid tranexamic acid ionic salt is of an orthorhombic crystal system.

By way of example, the supramolecular 4-methoxysalicylic acid tranexamic acid ionic salt has a P2₁2₁2₁ space group, and unit cell parameters of a = 7.9541(6) Å, b = 12.9509(12) Å, c = 16.5051(13) Å, α = 90°, β = 90°, γ = 90°, Z (the number of units in the unit cell) = 4, and a unit cell volume of 1700.2(2) Å³.

The present invention provides a preparation method for the supramolecular 4-methoxysalicylic acid tranexamic acid ionic salt provided above, the preparation method comprising: subjecting a liquid phase containing 4-methoxysalicylic acid, tranexamic acid, and an organic solvent to a heating and stirring treatment and an ultrasonic treatment in sequence, to obtain a supramolecular 4-methoxysalicylic acid tranexamic acid ionic salt solution; and subjecting the supramolecular 4-methoxysalicylic acid tranexamic acid ionic salt solution to a crystallization treatment.

In the preparation method for the supramolecular 4-methoxysalicylic acid tranexamic acid ionic salt provided by the present invention, 4-methoxysalicylic acid and tranexamic acid are used for reaction, the hydrogen atom of the 4-methoxysalicylic acid is transferred to the tranexamic acid, and proton transfer occurs, and then the 4-methoxysalicylic acid structure and the tranexamic acid structure are constructed to form a novel compound, i.e., a supramolecular 4-methoxysalicylic acid tranexamic acid ionic salt. The supramolecular 4-methoxysalicylic acid tranexamic acid ionic salt can not only effectively solve the problem of poor transdermal permeability of potassium 4-methoxysalicylate, but also effectively reduce the toxicity and side effects and irritation of potassium 4-methoxysalicylate and tranexamic acid when used alone, thereby expanding the application range thereof to a great extent. In addition, the 4-methoxysalicylic acid structure and the tranexamic acid structure in the supramolecular 4-methoxysalicylic acid tranexamic acid ionic salt have a synergistic effect, and compared with potassium 4-methoxysalicylate used alone or tranexamic acid used alone, the supramolecular 4-methoxysalicylic acid tranexamic acid ionic salt can effectively enhance the whitening effect, brightening effect, spot-fading effect, anti-yellowing effect, acne mark fading effect, and transdermal effect.

In addition, the preparation method for the supramolecular 4-methoxysalicylic acid tranexamic acid ionic salt provided by the present invention is simple and easy to implement, and can improve the preparation efficiency of the supramolecular 4-methoxysalicylic acid tranexamic acid ionic salt.

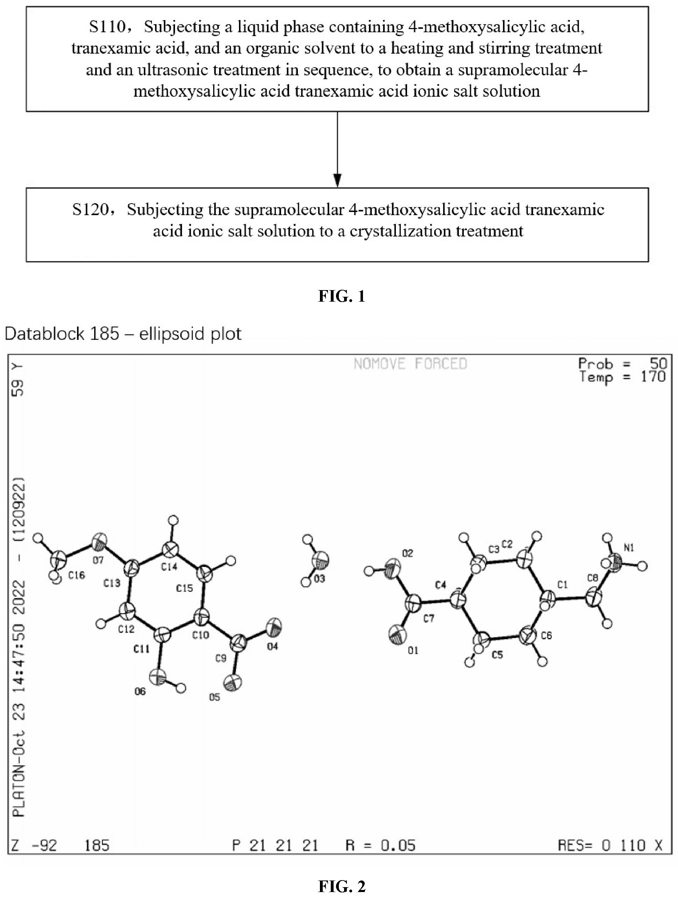

FIG. 1 is a flow chart of the preparation method for the supramolecular 4-methoxysalicylic acid tranexamic acid ionic salt provided by the present invention. Referring to FIG. 1, the preparation method for the supramolecular 4-methoxysalicylic acid tranexamic acid ionic salt comprises the following steps.

S110, subjecting a liquid phase containing 4-methoxysalicylic acid, tranexamic acid, and an organic solvent to a heating and stirring treatment and an ultrasonic treatment in sequence, to obtain a supramolecular 4-methoxysalicylic acid tranexamic acid ionic salt solution.

In some optional embodiments of the present invention, the preparation method for the liquid phase containing 4-methoxysalicylic acid, tranexamic acid, and an organic solvent comprises: dispersing the 4-methoxysalicylic acid and the tranexamic acid in an organic solvent respectively to obtain a 4-methoxysalicylic acid dispersion and a tranexamic acid dispersion, and then adding the 4-methoxysalicylic acid dispersion dropwise to the tranexamic acid dispersion to obtain a mixed solution as the liquid phase.

In the present invention, the mixing uniformity of the liquid phase can be improved by means of dispersing first and then mixing.

In some optional embodiments of the present invention, the molar ratio of the 4-methoxysalicylic acid to the tranexamic acid is (3 to 1): (1 to 3). With the above proportion, the reaction can be fully carried out, thereby facilitating improving the yield and purity of the prepared supramolecular 4-methoxysalicylic acid tranexamic acid ionic salt.

By way of example, the molar ratio of the 4-methoxysalicylic acid to the tranexamic acid can be any point value among 1:3, 1:2, 1:1, 2:1, and 3:1 or a range value between any two thereof.

Optionally, in some optional embodiments of the present invention, the molar ratio of the 4-methoxysalicylic acid to the tranexamic acid is 1:1. With the above proportion, the 4-methoxysalicylic acid and the tranexamic acid can more fully react, thereby further facilitating improving the yield and purity of the prepared supramolecular 4-methoxysalicylic acid tranexamic acid ionic salt.

In some optional embodiments of the present invention, the organic solvent comprises but is not limited to at least one of methanol, ethanol, isopropanol, N-methylpyrrolidone, tetrahydrofuran, benzene, toluene, petroleum ether, n-hexane, acetone, dioxane, N,N-dimethylformamide, ethyl acetate, and acetonitrile.

As a typical but non-limiting example, the organic solvent is selected from ethanol.

In some optional embodiments of the present invention, the temperature of the heating and stirring treatment is 80 °C to 110 °C. When the temperature of the heating and stirring treatment is within the above temperature range, the 4-methoxysalicylic acid and the tranexamic acid can be fully combined and the reaction is more thorough, which is beneficial to improving the conversion rate of the reaction between the 4-methoxysalicylic acid and the tranexamic acid, thereby facilitating improving the yield and purity of the prepared supramolecular 4-methoxysalicylic acid tranexamic acid ionic salt.

By way of example, the temperature of the heating and stirring treatment can be, for example, any point value among 80 °C, 85 °C, 90 °C, 95 °C, 100 °C, 105 °C, and 110 °C or a range value between any two thereof.

In some optional embodiments of the present invention, the heating and stirring treatment adopts oil bath heating.

In some optional embodiments of the present invention, the duration of the heating and stirring treatment is 10 h to 25 h. When the duration of the heating and stirring treatment is within the above time range, the 4-methoxysalicylic acid and the tranexamic acid can be fully combined and the reaction is more thorough, which is beneficial to improving the conversion rate of the reaction between the 4-methoxysalicylic acid and the tranexamic acid, thereby facilitating improving the yield and purity of the prepared supramolecular 4-methoxysalicylic acid tranexamic acid ionic salt.

By way of example, the duration of the heating and stirring treatment can be, for example, any point value among 10 h, 12 h, 14 h, 18 h, 20 h, 22 h, and 25 h or a range value between any two thereof.

In the present invention, the ultrasonic treatment can accelerate the reaction rate of the 4-methoxysalicylic acid and the tranexamic acid, so that the 4-methoxysalicylic acid anion and the tranexamic acid cation are effectively bound through an ionic bond.

In some optional embodiments of the present invention, the temperature of the ultrasonic treatment is 40 °C to 60 °C. Under the above ultrasonic treatment temperature, the 4-methoxysalicylic acid and the tranexamic acid can be further fully combined and the reaction is more thorough, which is beneficial to further improving the conversion rate of the reaction between the 4-methoxysalicylic acid and the tranexamic acid, thereby facilitating further improving the yield and purity of the prepared supramolecular 4-methoxysalicylic acid tranexamic acid ionic salt.

By way of example, the temperature of the ultrasonic treatment can be, for example, any point value among 40 °C, 45 °C, 50 °C, 55 °C, and 60 °C or a range value between any two thereof.

In some optional embodiments of the present invention, the ultrasonic frequency of the ultrasonic treatment is 20 kHz to 60 kHz. Under the above ultrasonic frequency of the ultrasonic treatment, the 4-methoxysalicylic acid and the tranexamic acid can be further fully combined and the reaction is more thorough, which is beneficial to further improving the conversion rate of the reaction between the 4-methoxysalicylic acid and the tranexamic acid, thereby facilitating further improving the yield and purity of the prepared supramolecular 4-methoxysalicylic acid tranexamic acid ionic salt.

By way of example, the ultrasonic frequency of the ultrasonic treatment can be, for example, any point value among 20 kHz, 25 kHz, 30 kHz, 35 kHz, 40 kHz, 45 kHz, 50 kHz, 55 kHz, and 60 kHz or a range value between any two thereof.

In some optional embodiments of the present invention, the ultrasonic power of the ultrasonic treatment is 280 W to 320 W. Under the above ultrasonic power of the ultrasonic treatment, the 4-methoxysalicylic acid and the tranexamic acid can be further fully combined and the reaction is more thorough, which is beneficial to further improving the conversion rate of the reaction between the 4-methoxysalicylic acid and the tranexamic acid, thereby facilitating further improving the yield and purity of the prepared supramolecular 4-methoxysalicylic acid tranexamic acid ionic salt.

By way of example, the ultrasonic power of the ultrasonic treatment can be, for example, any point value among 280 W, 285 W, 290 W, 295 W, 300 W, 305 W, 310 W, 315 W, and 320 W or a range value between any two thereof.

In some optional embodiments of the present invention, the duration of the ultrasonic treatment is 4 h to 8 h. Under the above duration of the ultrasonic treatment, the 4-methoxysalicylic acid and the tranexamic acid can be further fully combined and the reaction is more thorough, which is beneficial to further improving the conversion rate of the reaction between the 4-methoxysalicylic acid and the tranexamic acid, thereby facilitating further improving the yield and purity of the prepared supramolecular 4-methoxysalicylic acid tranexamic acid ionic salt.

By way of example, the duration of the ultrasonic treatment can be, for example, any point value among 4 h, 5 h, 6 h, 7 h, and 8 h or a range value between any two thereof.

In some optional embodiments of the present invention, the preparation method for the supramolecular 4-methoxysalicylic acid tranexamic acid ionic salt solution further comprises: after the ultrasonic treatment, subjecting the system after the ultrasonic treatment to a concentration treatment. The above manner facilitates better performing the subsequent crystallization treatment and facilitates the precipitation of crystals.

By way of example, the concentration treatment can adopt a reduced-pressure concentration manner.

In some optional embodiments of the present invention, the system after the ultrasonic treatment is subjected to a concentration treatment, so as to obtain a supersaturated supramolecular 4-methoxysalicylic acid tranexamic acid ionic salt solution. The above manner further facilitates the rapid precipitation of crystals subsequently.

In some optional embodiments of the present invention, the preparation process of the supramolecular 4-methoxysalicylic acid tranexamic acid ionic salt solution is carried out under an inert gas atmosphere. The inert gas atmosphere is beneficial to preventing the 4-methoxysalicylic acid and the tranexamic acid from being oxidized during the reaction process, and is beneficial to preventing the biological activities of the 4-methoxysalicylic acid and the tranexamic acid from being destroyed, thereby improving the yield and purity of the prepared supramolecular 4-methoxysalicylic acid tranexamic acid ionic salt, and also facilitating fully exerting the whitening effect, brightening effect, spot-fading effect, anti-yellowing effect, acne mark fading effect, and transdermal effect.

Optionally, the inert gas comprises, but is not limited to, at least one of nitrogen, helium, neon, and argon.

In some optional embodiments of the present invention, the preparation method for the supramolecular 4-methoxysalicylic acid tranexamic acid ionic salt solution comprises: dissolving 4-methoxysalicylic acid in the aforementioned organic solvent to obtain a first system, and dissolving tranexamic acid in the aforementioned organic solvent to obtain a second system; and then, adding the first system dropwise to the second system or adding the second system dropwise to the first system, to obtain the aforementioned "liquid phase containing 4-methoxysalicylic acid, tranexamic acid, and an organic solvent".

S120, subjecting the supramolecular 4-methoxysalicylic acid tranexamic acid ionic salt solution to a crystallization treatment.

In some optional embodiments of the present invention, the crystallization treatment adopts a cooling crystallization manner.

Optionally, the temperature of the crystallization treatment is ≤ 0 °C; under the above crystallization temperature, the supramolecular 4-methoxysalicylic acid tranexamic acid ionic salt solution can be fully crystallized to precipitate crystals, which is beneficial to improving the yield and purity of the prepared supramolecular 4-methoxysalicylic acid tranexamic acid ionic salt.

By way of example, the temperature of the crystallization treatment is -20 °C.

In some optional embodiments of the present invention, the crystallization treatment is carried out under a vacuum condition; and under the above crystallization treatment conditions, the supramolecular 4-methoxysalicylic acid tranexamic acid ionic salt solution can be fully crystallized to precipitate crystals, which is beneficial to improving the yield and purity of the prepared supramolecular 4-methoxysalicylic acid tranexamic acid ionic salt.

In some optional embodiments of the present invention, the preparation method for the supramolecular 4-methoxysalicylic acid tranexamic acid ionic salt further comprises: after the crystallization treatment, sequentially filtering, washing, and drying the system after the crystallization treatment.

By way of example, the washing of the system after the crystallization treatment can adopt at least one of methanol, ethanol, isopropanol, N-methylpyrrolidone, tetrahydrofuran, benzene, toluene, petroleum ether, n-hexane, acetone, dioxane, N,N-dimethylformamide, ethyl acetate, and acetonitrile.

In some optional embodiments of the present invention, the preparation process of the supramolecular 4-methoxysalicylic acid tranexamic acid ionic salt is carried out under an inert gas atmosphere; the inert gas atmosphere is beneficial to preventing the 4-methoxysalicylic acid and the tranexamic acid from being oxidized during the reaction process, and is beneficial to preventing the biological activities of the 4-methoxysalicylic acid and the tranexamic acid from being destroyed, thereby improving the yield and purity of the prepared supramolecular 4-methoxysalicylic acid tranexamic acid ionic salt, and also facilitating fully exerting the whitening effect, brightening effect, spot-fading effect, anti-yellowing effect, acne mark fading effect, and transdermal effect. During the preparation process of the supramolecular 4-methoxysalicylic acid tranexamic acid ionic salt, an inert gas can also be slowly supplemented to maintain a constant temperature and a constant pressure.

Optionally, the inert gas comprises, but is not limited to, at least one of nitrogen, helium, neon, and argon.

The present invention provides use of the supramolecular 4-methoxysalicylic acid tranexamic acid ionic salt provided above in the preparation of a drug, a cosmetic, or a skincare product.

The supramolecular 4-methoxysalicylic acid tranexamic acid ionic salt provided by the present invention can not only effectively solve the problem of poor transdermal permeability of potassium 4-methoxysalicylate, but also effectively reduce the toxicity and side effects and irritation of potassium 4-methoxysalicylate and tranexamic acid when used alone, thereby expanding the application range thereof to a great extent. In addition, the 4-methoxysalicylic acid structure and the tranexamic acid structure in the supramolecular 4-methoxysalicylic acid tranexamic acid ionic salt have a synergistic effect, and compared with potassium 4-methoxysalicylate used alone or tranexamic acid used alone, the supramolecular 4-methoxysalicylic acid tranexamic acid ionic salt can effectively enhance the whitening effect, brightening effect, spot-fading effect, anti-yellowing effect, acne mark fading effect, and transdermal effect. Therefore, the supramolecular 4-methoxysalicylic acid tranexamic acid ionic salt can be used in the preparation of drugs, cosmetics, and skincare products having the effects of whitening, spot fading, acne mark fading, etc.

In some optional embodiments of the present invention, the aforementioned drug, cosmetic, and skincare product can comprise an aqueous solution of the supramolecular 4-methoxysalicylic acid tranexamic acid ionic salt. By way of example, the aqueous solution of the supramolecular 4-methoxysalicylic acid tranexamic acid ionic salt can be prepared by mixing deionized water and the supramolecular 4-methoxysalicylic acid tranexamic acid ionic salt. The mass percentage of the supramolecular 4-methoxysalicylic acid tranexamic acid ionic salt in the drug, the cosmetic, or the skincare product is 1%-5%, and by way of example, the mass percentage of the supramolecular 4-methoxysalicylic acid tranexamic acid ionic salt in the drug, the cosmetic, or the skincare product can be, for example, any point value among 1%, 2%, 3%, 4%, and 5% or a range value between any two thereof.

In order to make the purposes, technical solutions, and advantages of the embodiments of the present invention clearer, the technical solutions in the embodiments of the present invention will be described clearly and completely below. If no specific conditions are indicated in the examples, the conventional conditions or the conditions recommended by the manufacturer are used. If the reagents or instruments used are not indicated with manufacturers, they are all conventional products that can be purchased commercially.

### Example 1

This example provides a supramolecular 4-methoxysalicylic acid tranexamic acid ionic salt, and the preparation method thereof comprises the following steps.

1 mol of 4-methoxysalicylic acid and 1 mol of tranexamic acid were respectively dispersed in an ethanol solution to obtain a 4-methoxysalicylic acid ethanol dispersion and a tranexamic acid ethanol dispersion, respectively, and the 4-methoxysalicylic acid ethanol dispersion was slowly added dropwise to the tranexamic acid ethanol dispersion to obtain a mixed solution. The mixed solution was placed in a reaction kettle; under the protection of an argon atmosphere, after being stirred in an oil bath at 80 °C for 24 h, an ultrasonic reaction was carried out for 5 h under the condition of an ultrasonic input power of 300 W, an ultrasonic frequency of 40 kHz, and an ultrasonic temperature of 40 °C; as the reaction proceeded, argon was slowly supplemented to ensure that the temperature and the pressure in the kettle were maintained constant, and then the reaction solution was concentrated under reduced pressure to obtain a supersaturated solution. The supersaturated solution was placed at a temperature of - 20 °C for a low-temperature crystallization treatment, and the precipitated ionic salt compound was filtered and washed 3 times with a frozen ethanol solvent, and dried in a vacuum drying oven at 60 °C, to obtain the supramolecular 4-methoxysalicylic acid tranexamic acid ionic salt.

### Example 2

This example provides a supramolecular 4-methoxysalicylic acid tranexamic acid ionic salt, and the preparation method thereof comprises the following steps.

1 mol of 4-methoxysalicylic acid and 1 mol of tranexamic acid were respectively dispersed in an ethanol solution to obtain a 4-methoxysalicylic acid ethanol dispersion and a tranexamic acid ethanol dispersion, respectively, and the 4-methoxysalicylic acid ethanol dispersion was slowly added dropwise to the tranexamic acid ethanol dispersion to obtain a mixed solution. The mixed solution was placed in a reaction kettle; under the protection of an argon atmosphere, after being stirred in an oil bath at 80 °C for 12 h, an ultrasonic reaction was carried out for 5 h under the conditions of an ultrasonic input power of 300 W, an ultrasonic frequency of 40 kHz, and an ultrasonic temperature of 50 °C; as the reaction proceeded, argon was slowly supplemented to ensure that the temperature and the pressure in the kettle were maintained constant, and then the reaction solution was concentrated under reduced pressure to obtain a supersaturated solution. The supersaturated solution was placed at a temperature of - 20 °C for a low-temperature crystallization treatment, and the precipitated ionic salt compound was filtered and washed 3 times with a frozen ethanol solvent, and dried in a vacuum drying oven at 60 °C, to obtain the supramolecular 4-methoxysalicylic acid tranexamic acid ionic salt.

### Example 3

This example provides a supramolecular 4-methoxysalicylic acid tranexamic acid ionic salt, and the preparation method thereof comprises the following steps.

1 mol of 4-methoxysalicylic acid and 2 mol of tranexamic acid were respectively dispersed in an ethanol solution to obtain a 4-methoxysalicylic acid ethanol dispersion and a tranexamic acid ethanol dispersion, respectively, and the 4-methoxysalicylic acid ethanol dispersion was slowly added dropwise to the tranexamic acid ethanol dispersion to obtain a mixed solution. The mixed solution was placed in a reaction kettle; under the protection of an argon atmosphere, after being stirred in an oil bath at 90 °C for 12 h, an ultrasonic reaction was carried out for 5 h under the conditions of an ultrasonic input power of 300 W, an ultrasonic frequency of 40 kHz, and an ultrasonic temperature of 50 °C; as the reaction proceeded, argon was slowly supplemented to ensure that the temperature and the pressure in the kettle were maintained constant, and then the reaction solution was concentrated under reduced pressure to obtain a supersaturated solution. The supersaturated solution was placed at a temperature of - 20 °C for a low-temperature crystallization treatment, and the precipitated ionic salt compound was filtered and washed 3 times with a frozen ethanol solvent, and dried in a vacuum drying oven at 60 °C, to obtain the supramolecular 4-methoxysalicylic acid tranexamic acid ionic salt.

### Comparative Example 1

This comparative example uses potassium 4-methoxysalicylate.

### Comparative Example 2

This comparative example uses tranexamic acid.

### Comparative Example 3

This comparative example uses a mixture of potassium 4-methoxysalicylate and tranexamic acid with a molar ratio of 1:1.

### Experimental Example 1

The melting point of the supramolecular 4-methoxysalicylic acid tranexamic acid ionic salt obtained in Example 1 was tested, and the melting point thereof is 185 °C to 190 °C, which meets the conditions of an ionic salt.

An X-ray diffractometer (SuperNova, Dual, Mo at zero, AtlasS2 diffractometer) was used to perform an X-ray single crystal diffraction test on the supramolecular 4-methoxysalicylic acid tranexamic acid ionic salt obtained in Example 1, the test temperature being 170.00(10) K, and Olex2 and ShelXL were used for structural analysis. The molecular structure diagram of the supramolecular 4-methoxysalicylic acid tranexamic acid ionic salt prepared in Example 1 is shown in FIG. 2, and the X-ray single crystal diffraction test results of the supramolecular 4-methoxysalicylic acid tranexamic acid ionic salt prepared in Example 1 are shown in Table 1.

**Table 1 Single crystal data of the supramolecular 4-methoxysalicylic acid tranexamic acid ionic salt prepared in Example 1**

| Chemical formula | C₁₆H₂₅NO₇ | | Absorption coefficient/mm⁻¹ | 0.884 |
|---|---|---|---|---|
| Molecular weight | 343.37 | | F(0000) | 736.0 |
| Temperature /K | 169.99(10) | | Crystal size/mm³ | 0.14×0.12×0.1 |
| Crystal system | orthorhombic | | Radiation | Cu Kα (λ=1.54184) |
| Space group | P2₁2₁2₁ | | Data collection angle range (2θ)/° | 8.678 to 148.628 |
| Unit cell parameters | a/Å | 7.9541(6) | Index ranges | -8≤h≤9, -16≤k≤16, -19≤l≤20 |
| | b/Å | 12.9509(12) | Reflections collected | 7425 |
| | c/Å | 16.5051(13) | Independent reflections | 3316[Rᵢₙₜ=0.0481, R_{sigma}=0.0470] |
| | α/° | 90 | Data/restraints/para meters | 3316/0/225 |
| | β/° | 90 | F² goodness-of-fit | 1.173 |
| | γ/° | 90 | Final R values (intensity > 2σ) | R₁=0.0546, wR₂=0.1567 |
| Volume/Å³ | 1700.2(2) | | R values (all data) | R₁=0.0606, wR₂=0.1695 |
| Unit number in the unit cell | 4 | | Largest difference peak/hole/e Å⁻³ | 0.23/-0.24 |
| Density (calculated)/ g/cm³ | 1.341 | | | |

The atomic coordinates (×10⁴) and the equivalent isotropic atomic displacement parameters (Å²×10³) of the single crystal of the supramolecular 4-methoxysalicylic acid tranexamic acid ionic salt prepared in Example 1 are shown in Table 2, where U(eq) is defined as one third of the trace of the orthogonal Uᵢⱼ tensor.

**Table 2 Atomic coordinates and equivalent isotropic atomic displacement parameters of the single crystal of the supramolecular 4-methoxysalicylic acid tranexamic acid ionic salt prepared in Example 1**

| Atom | x | y | z | U(eq) |
|---|---|---|---|---|
| O1 | 4053(5) | 3652(3) | 3939.5(18) | 49.7(8) |
| O2 | 4755(5) | 5184(2) | 4433.9(17) | 54.5(9) |
| N1 | 5623(5) | 6711(3) | 248.8(18) | 35.3(8) |
| C1 | 4455(5) | 5599(3) | 1345(2) | 31.9(8) |
| C2 | 4999(5) | 6353(3) | 2013(2) | 33.3(9) |
| C3 | 5335(5) | 5768(3) | 2804(2) | 32.9(8) |
| C4 | 3791(5) | 5157(3) | 3083(2) | 32.5(8) |
| C5 | 3182(6) | 4428(4) | 2416(2) | 39.6(10) |
| C6 | 2883(5) | 5008(4) | 1621(2) | 37.8(9) |
| C7 | 4209(5) | 4576(3) | 3851(2) | 34.3(9) |
| C8 | 4122(5) | 6131(4) | 539(2) | 38.6(10) |
| O3 | 6286(4) | 4351(2) | 5691.3(15) | 37.3(7) |
| O4 | 4816(4) | 2660(2) | 6231.8(15) | 40.2(7) |
| O5 | 3645(4) | 1251(2) | 6729.0(16) | 41.2(7) |
| O6 | 3409(4) | 926(2) | 8190.5(16) | 41.9(7) |
| O7 | 5403(4) | 3506(2) | 10034.8(14) | 38.3(7) |
| C9 | 4360(5) | 2123(3) | 6826(2) | 31.7(8) |
| C10 | 4619(5) | 2479(3) | 7672(2) | 29.7(8) |
| C11 | 4140(5) | 1860(3) | 8327(2) | 30.7(8) |
| C12 | 4379(5) | 2170(3) | 9125(2) | 35.5(9) |
| C13 | 5113(5) | 3129(3) | 9274(2) | 31.5(8) |
| C14 | 5596(5) | 3765(3) | 8635(2) | 31.9(8) |
| C15 | 5359(5) | 3439(3) | 7851(2) | 31.6(8) |
| C16 | 5035(6) | 2833(3) | 10703(2) | 40.8(10) |

The analysis data of anisotropic atomic displacement parameter of the single crystal of the supramolecular 4-methoxysalicylic acid tranexamic acid ionic salt prepared in Example 1 are shown in Table 3, where the exponential expression of the anisotropic atomic displacement factor takes the form: 2π2[h2a*2U11+2hka*b*U12+...].

**Table 3 Anisotropic atomic displacement parameters of the single crystal of the supramolecular 4-methoxysalicylic acid tranexamic acid ionic salt prepared in Example 1**

| Atom | U11 | U22 | U33 | U23 | U13 | U12 |
|---|---|---|---|---|---|---|
| O1 | 75(2) | 37.8(19) | 36.4(15) | 6.2(13) | -5.8(15) | -7.1(17) |
| 02 | 91(3) | 42.5(19) | 30.0(14) | 4.2(13) | -16.7(16) | -7.3(19) |
| N1 | 44.3(19) | 36.3(19) | 25.4(14) | 2.6(14) | -0.2(14) | 2.1(16) |
| C1 | 33.2(19) | 36(2) | 26.5(16) | 2.9(15) | -2.5(15) | 4.1(17) |
| C2 | 35.6(19) | 38(2) | 26.4(16) | 1.6(16) | -1.2(15) | 1.8(17) |
| C3 | 30.8(18) | 42(2) | 26.3(16) | -2.3(16) | -1.1(14) | -3.3(17) |
| C4 | 34.0(19) | 36(2) | 27.1(16) | 1.5(16) | -0.6(14) | -0.4(16) |
| C5 | 42(2) | 46(3) | 30.5(18) | 1.3(18) | -1.6(17) | -14.0(19) |
| C6 | 34.6(19) | 48(3) | 31.2(19) | 0.7(19) | -1.8(15) | -5.7(19) |
| C7 | 39(2) | 37(2) | 27.0(17) | -0.8(16) | 4.8(16) | -2.1(17) |
| C8 | 39(2) | 49(3) | 27.8(17) | 2.8(18) | -3.3(15) | -0.2(19) |
| 03 | 44.7(16) | 38.2(17) | 29.0(13) | -0.9(12) | 3.2(12) | -4.2(13) |
| 04 | 57.5(19) | 39.9(17) | 23.2(12) | 0.1(11) | -0.5(12) | -3.7(14) |
| 05 | 57.5(18) | 33.5(16) | 32.7(13) | -2.6(12) | -5.6(13) | -8.6(14) |
| 06 | 62.0(19) | 33.5(16) | 30.3(13) | 1.0(12) | -3.7(13) | -14.6(15) |
| 07 | 54.0(17) | 39.2(17) | 21.7(12) | -1.9(11) | -0.8(12) | -5.7(14) |
| C9 | 34.8(19) | 32(2) | 28.1(17) | -2.0(16) | -1.1(15) | 3.5(16) |
| C10 | 28.4(17) | 35(2) | 25.3(16) | 1.6(15) | 1.0(13) | 1.1(16) |
| C11 | 33.3(18) | 28.9(19) | 29.8(17) | -2.9(15) | -0.9(15) | -4.1(16) |
| C12 | 40(2) | 39(2) | 28.3(17) | 3.2(16) | 1.4(16) | -5.3(18) |
| C13 | 34.3(18) | 38(2) | 22.4(16) | -1.3(15) | 0.6(14) | -1.8(16) |
| C14 | 34.2(19) | 33(2) | 28.0(17) | -0.9(15) | -1.5(15) | -4.9(16) |
| C15 | 32.7(18) | 34(2) | 27.8(16) | 3.5(15) | 2.2(14) | -3.3(16) |
| C16 | 56(3) | 40(2) | 26.2(17) | 0.4(17) | 1.6(18) | -8(2) |

The bond length analysis data of each chemical bond of the single crystal of the supramolecular 4-methoxysalicylic acid tranexamic acid ionic salt prepared in Example 1 are shown in Table 4.

**Table 4 Bond length of each chemical bond of the single crystal of the supramolecular 4-methoxysalicylic acid tranexamic acid ionic salt prepared in Example 1**

| Atom | Atom | Bond length/Å |
|---|---|---|
| O1 | C7 | 1.212(5) |
| O2 | C7 | 1.316(5) |
| N1 | C8 | 1.489(5) |
| C1 | C2 | 1.535(5) |
| C1 | C6 | 1.535(5) |
| C1 | C8 | 1.522(5) |
| C2 | C3 | 1.532(5) |
| C3 | C4 | 1.532(5) |
| C4 | C5 | 1.529(5) |
| C4 | C7 | 1.511(5) |
| C5 | C6 | 1.530(6) |
| O4 | C9 | 1.256(5) |
| O5 | C9 | 1.275(5) |
| O6 | C11 | 1.361(5) |
| O7 | C13 | 1.367(4) |
| O7 | C16 | 1.435(5) |
| C9 | C10 | 1.484(5) |
| C10 | C11 | 1.399(5) |
| C10 | C15 | 1.407(5) |
| C11 | C12 | 1.390(5) |
| C12 | C13 | 1.394(6) |
| C13 | C14 | 1.392(5) |
| C14 | C15 | 1.375(5) |

The bond angle analysis data of each chemical bond of the single crystal of the supramolecular 4-methoxysalicylic acid tranexamic acid ionic salt prepared in Example 1 are shown in Table 5.

**Table 5 Bond angle of each chemical bond of the single crystal of the supramolecular 4-methoxysalicylic acid tranexamic acid ionic salt prepared in Example 1**

| Atom | Atom | Atom | Bond angle/° |
|---|---|---|---|
| C6 | C1 | C2 | 109.5(3) |
| C8 | C1 | C2 | 112.9(3) |
| C8 | C1 | C6 | 110.1(3) |
| C3 | C2 | C1 | 110.3(3) |
| C4 | C3 | C2 | 111.8(3) |
| C5 | C4 | C3 | 110.9(3) |
| C7 | C4 | C3 | 109.5(3) |
| C7 | C4 | C5 | 111.5(3) |
| C4 | C5 | C6 | 111.3(4) |
| C5 | C6 | C1 | 111.9(3) |
| O1 | C7 | O2 | 122.4(4) |
| O1 | C7 | C4 | 124.7(4) |
| O2 | C7 | C4 | 112.8(4) |
| N1 | C8 | C1 | 111.7(3) |
| C13 | O7 | C16 | 117.1(3) |
| O4 | C9 | O5 | 121.4(3) |
| O4 | C9 | C10 | 121.5(4) |
| O5 | C9 | C10 | 117.1(3) |
| C11 | C10 | C9 | 120.8(3) |
| C11 | C10 | C15 | 117.2(3) |
| C15 | C10 | C9 | 122.0(3) |
| O6 | C11 | C10 | 119.8(3) |
| O6 | C11 | C12 | 118.1(4) |
| C12 | C11 | C10 | 122.0(4) |
| C11 | C12 | C13 | 118.8(4) |
| O7 | C13 | C12 | 123.4(3) |
| O7 | C13 | C14 | 116.0(3) |
| C14 | C13 | C12 | 120.7(3) |
| C15 | C14 | C13 | 119.6(4) |
| C14 | C15 | C10 | 121.8(3) |

The hydrogen bond analysis data of the single crystal of the supramolecular 4-methoxysalicylic acid tranexamic acid ionic salt prepared in Example 1 are shown in Table 6.

**Table 6 Hydrogen bonds of the single crystal of the supramolecular 4-methoxysalicylic acid tranexamic acid ionic salt prepared in Example 1**

| D | H | A | d(D-H)/Å | d(H-A)/Å | d(D-A)/Å | D-H-A/° |
|---|---|---|---|---|---|---|
| O2 | H2 | O3 | 0.84 | 1.82 | 2.637(4) | 164.2 |
| N1 | H1A | O41 | 0.91 | 1.88 | 2.758(4) | 160.5 |
| N1 | H1C | O11 | 0.91 | 1.95 | 2.861(5) | 173.6 |
| O3 | H3C | O4 | 0.85 | 1.79 | 2.638(4) | 171.0 |
| O3 | H3D | 062 | 0.85 | 1.92 | 2.761(4) | 172.4 |
| O6 | H6 | 05 | 0.84 | 1.70 | 2.456(4) | 149.1 |

The torsion angle analysis data of each chemical bond of the single crystal of the supramolecular 4-methoxysalicylic acid tranexamic acid ionic salt prepared in Example 1 are shown in Table 7.

**Table 7 Torsion angle of each chemical bond of the single crystal of the supramolecular 4-methoxysalicylic acid tranexamic acid ionic salt prepared in Example 1**

| A | B | C | D | Angle/° |
|---|---|---|---|---|
| C1 | C2 | C3 | C4 | -57.5(4) |
| C2 | C1 | C6 | C5 | -57.4(4) |
| C2 | C1 | C8 | N1 | 57.5(5) |
| C2 | C3 | C4 | C5 | 55.1(5) |
| C2 | C3 | C4 | C7 | 178.5(3) |
| C3 | C4 | C5 | C6 | -53.6(5) |
| C3 | C4 | C7 | O1 | -124.5(5) |
| C3 | C4 | C7 | 02 | 56.2(5) |
| C4 | C5 | C6 | C1 | 55.7(5) |
| C5 | C4 | C7 | O1 | -1.4(6) |
| C5 | C4 | C7 | 02 | 179.3(4) |
| C6 | C1 | C2 | C3 | 57.7(4) |
| C6 | C1 | C8 | N1 | -179.8(3) |
| C7 | C4 | C5 | C6 | -175.9(3) |
| C8 | C1 | C2 | C3 | -179.3(3) |
| C8 | C1 | C6 | C5 | 178.0(4) |
| 04 | C9 | C10 | C11 | -178.4(4) |
| 04 | C9 | C10 | C15 | 1.1(6) |
| 05 | C9 | C10 | C11 | 2.0(5) |
| 05 | C9 | C10 | C15 | -178.5(4) |
| 06 | C11 | C12 | C13 | -179.5(4) |
| 07 | C13 | C14 | C15 | 179.5(4) |
| C9 | C10 | C11 | 06 | -0.8(6) |
| C9 | C10 | C11 | C12 | 179.5(4) |
| C9 | C10 | C15 | C14 | 180.0(4) |
| C10 | C11 | C12 | C13 | 0.2(6) |
| C11 | C10 | C15 | C14 | -0.5(6) |
| C11 | C12 | C13 | O7 | -180.0(4) |
| C11 | C12 | C13 | C14 | 0.2(6) |
| C12 | C13 | C14 | C15 | -0.6(6) |
| C13 | C14 | C15 | C10 | 0.8(6) |
| C15 | C10 | C11 | 06 | 179.6(3) |
| C15 | C10 | C11 | C12 | 0.0(6) |
| C16 | 07 | C13 | C12 | 4.8(6) |
| C16 | 07 | C13 | C14 | -175.4(4) |

The hydrogen atom coordinates (Å×10⁴) and the isotropic atomic displacement parameters (Å×10³) of the single crystal of the supramolecular 4-methoxysalicylic acid tranexamic acid ionic salt prepared in Example 1 are shown in Table 8.

**Table 8 Hydrogen atom coordinates and isotropic atomic displacement parameters of the single crystal of the supramolecular 4-methoxysalicylic acid tranexamic acid ionic salt prepared in Example 1**

| Atom | x | y | z | U(eq) |
|---|---|---|---|---|
| H2 | 5121.8 | 4823.05 | 4817.97 | 82 |
| H1A | 5489.19 | 6872.59 | -283.78 | 53 |
| H1B | 6556.85 | 6312.65 | 310.22 | 53 |
| H1C | 5738.72 | 7300.69 | 542.85 | 53 |
| H1 | 5381.51 | 5087.33 | 1263.07 | 38 |
| H2A | 6030.52 | 6720.46 | 1842.34 | 40 |
| H2B | 4103.77 | 6871.84 | 2101.41 | 40 |
| H3A | 6288.88 | 5288.39 | 2723.58 | 40 |
| H3B | 5655.51 | 6267.42 | 3231.27 | 40 |
| H4 | 2868.76 | 5657.34 | 3205.97 | 39 |
| H5A | 2123.19 | 4092.65 | 2589.9 | 47 |
| H5B | 4031.2 | 3880.3 | 2328.02 | 47 |
| H6A | 1943.8 | 5500.3 | 1693.99 | 45 |
| H6B | 2554.82 | 4508.61 | 1195.27 | 45 |
| H8A | 3805.24 | 5606.94 | 129.66 | 46 |
| H8B | 3165.86 | 6614.07 | 600.58 | 46 |
| H3C | 5714.98 | 3842.19 | 5864.86 | 56 |
| H3D | 6400.54 | 4793.61 | 6067.57 | 56 |
| H6 | 3315.74 | 828.99 | 7689.34 | 63 |
| H12 | 4046.85 | 1737.14 | 9561.2 | 43 |
| H14 | 6085.23 | 4420.6 | 8740.87 | 38 |
| H15 | 5704.63 | 3873.26 | 7417.89 | 38 |
| H16A | 5628.09 | 2176.45 | 10629.32 | 61 |
| H16B | 3821.76 | 2706.25 | 10726.96 | 61 |
| H16C | 5405.4 | 3157.62 | 11208.88 | 61 |

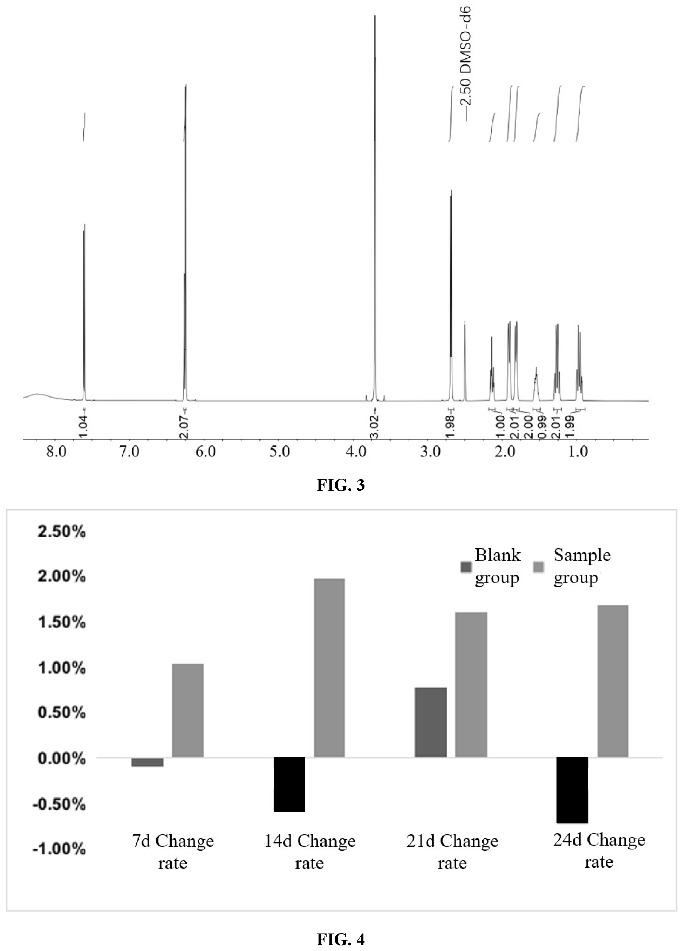

The supramolecular 4-methoxysalicylic acid tranexamic acid ionic salt prepared in Example 1 was characterized by ¹H NMR spectroscopy, and the ¹H NMR spectrum is shown in FIG. 3.

The ¹H NMR data of FIG. 3 are as follows:¹H NMR (600 MHz, DMSO-*d*₆) *δ* 7.61 (d, *J* = 8.3 Hz, 1H), 6.49 - 6.15 (m, 2H), 3.71 (s, 3H), 2.68 (d, *J* = 6.9 Hz, 2H), 2.13 (tt, *J* = 12.1, 3.6 Hz, 1H), 1.90 (dd, *J* = 13.6, 3.6 Hz, 2H), 1.81 (dd, *J* = 13.5, 3.6 Hz, 2H), 1.54 (dqd, *J* = 11.3, 7.3, 3.4 Hz, 1H), 1.26 (qd, *J* = 13.2, 3.4 Hz, 2H), 0.96 (qd, *J* = 13.1, 3.5 Hz, 2H).

As can be seen from FIG. 3, there are signals of 3 hydrogen atoms on the phenolic ring of the 4-methoxysalicylic acid and 1 methoxy substitution on the benzene ring, the hydrogen atom on the N of the tranexamic acid, 2 methine groups and 4 methylene groups on the six-membered ring, and one methylene group on the side chain; and the rest are a small amount of residual deuterated reagent peaks, and no obvious impurity peaks are observed. This shows that: in the supramolecular 4-methoxysalicylic acid tranexamic acid ionic salt prepared in Example 1, the 4-methoxysalicylic acid and the tranexamic acid exist in a molecular ratio of 1:1, and the purity thereof exceeds 98%.

Experimental Example 2 Cytotoxicity detection

The steps of the cytotoxicity detection are as follows.

### (1) Preparing test samples with different concentrations

Test sample 1: the supramolecular 4-methoxysalicylic acid tranexamic acid ionic salt obtained in Example 1 was dissolved in a Gibco cell culture medium and prepared into different concentrations, and the concentrations of the supramolecular 4-methoxysalicylic acid tranexamic acid ionic salt in the liquid phase were 0.0195 mg/mL, 0.0391 mg/mL, 0.0781 mg/mL, 0.1563 mg/mL, 0.3125 mg/mL, and 0.625 mg/mL, respectively.

Test sample 2: the tranexamic acid of Comparative Example 2 was dissolved in a Gibco cell culture medium and prepared into different concentrations, and the concentrations of the tranexamic acid in the liquid phase were 0.0195 mg/mL, 0.0391 mg/mL, 0.0781 mg/mL, 0.1563 mg/mL, 0.3125 mg/mL, and 0.625 mg/mL, respectively.

Test sample 3: the potassium 4-methoxysalicylate of Comparative Example 1 was dissolved in a Gibco cell culture medium and prepared into different concentrations, and the concentrations of the potassium 4-methoxysalicylate in the liquid phase were 0.0195 mg/mL, 0.0391 mg/mL, 0.0781 mg/mL, 0.1563 mg/mL, 0.3125 mg/mL, and 0.625 mg/mL, respectively.

### (2) Cell inoculation

Human melanocytes were inoculated at a seeding density of 1×10⁴ cells/well into a 96-well plate, and the cells were placed in an incubator (37 °C, 5% CO₂, 95% RH) for incubation overnight.

### (3) Administration

When the seeding efficiency of the cells in the 96-well plate reached 40% to 60%, administration was carried out; and after the administration was completed, the 96-well plate was placed in an incubator (37 °C, 5% CO₂, 95% RH) for culture.

200 µL of the cell culture medium was added to each well of the blank control group; 200 µL of the cell culture medium containing 10 wt% DMSO was added to each well of the positive control group; 200 µL of the test sample 1, the test sample 2, and the test sample 3 with different concentrations was added to each well of the test sample groups; and the zero-adjustment wells were not inoculated with cells, and only 200 µL of the cell culture medium was added. Three replicate wells were set for each concentration of each test sample.

### (4) Detection

After the cells were incubated and cultured for 24 h, the supernatant was discarded, an MTT working solution (0.5 mg/mL) was added, and the cells were incubated at 37 °C in the dark for 4 h; and after the incubation was completed, the supernatant was discarded, 150 µL of DMSO was added to each well, and the OD value was read at 490 nm.

The cell viability (%) = [(OD of the administered well - OD of the zero-adjustment well)/(OD of the blank control well - OD of the zero-adjustment well)] × 100%, wherein the administered well is the positive control group or the test sample group.

The cytotoxicity results of the test sample 1 with different concentrations are shown in Table 9.

**Table 9 Cytotoxicity of the test sample 1 with different concentrations**

| Cell viability (%) | Concentration gradient (mg/mL) | | | | | | |
|---|---|---|---|---|---|---|---|
| | 0.00 | 0.0195 | 0.0391 | 0.0781 | 0.1563 | 0.3125 | 0.625 |
| Mean | 100.00 | 102.68 | 100.36 | 96.94 | 95.46 | 94.16 | 88.49 |
| SD | 4.07 | 7.77 | 1.91 | 4.85 | 2.62 | 2.97 | 3.03 |
| P/value | / | 0.6676 | 0.8690 | 0.5780 | 0.2797 | 0.2620 | 0.0740 |

The cytotoxicity results of the test sample 2 with different concentrations are shown in Table 10.

**Table 10 Cytotoxicity of the test sample 2 with different concentrations**

| Cell viability (%) | Concentration gradient (mg/mL) | | | | | | |
|---|---|---|---|---|---|---|---|
| | 0.00 | 0.0195 | 0.0391 | 0.0781 | 0.1563 | 0.3125 | 0.625 |
| Mean | 100.00 | 98.12 | 92.93 | 91.13 | 92.77 | 90.45 | 87.33 |
| SD | 6.25 | 7.88 | 5.25 | 3.53 | 7.01 | 4.79 | 0.50 |
| P/value | / | 0.8088 | 0.3356 | 0.2202 | 0.4220 | 0.2439 | 0.0668 |

The cytotoxicity results of the test sample 3 with different concentrations are shown in Table 11.

**Table 11 Cytotoxicity of the test sample 3 with different concentrations**

| Cell viability (%) | Concentration gradient (mg/mL) | | | | | | |
|---|---|---|---|---|---|---|---|
| | 0.00 | 0.0195 | 0.0391 | 0.0781 | 0.1563 | 0.3125 | 0.625 |
| Mean | 100.00 | 95.25 | 94.25 | 90.10 | 85.17 | 82.32 | 80.43 |
| SD | 2.37 | 3.94 | 3.41 | 5.71 | 3.35 | 0.88 | 2.62 |
| P/value | / | 0.1675 | 0.2256 | 0.1036 | 0.0028 | 0.0026 | 0.0018 |

As can be seen from Table 9 to Table 11, the cytotoxicity of the supramolecular 4-methoxysalicylic acid tranexamic acid ionic salt of Example 1 is lower than that of the potassium 4-methoxysalicylate of Comparative Example 1 and the tranexamic acid of Comparative Example 2, indicating that the supramolecular 4-methoxysalicylic acid tranexamic acid ionic salt of Example 1 is more suitable for use on skin.

### Experimental Example 3 Melanin content detection

The steps of the melanin content detection are as follows.

### (1) Solution preparation and grouping

Blank control (BC): Gibco cell culture medium.

Positive control (PC): glabridin was dissolved in a cell culture medium, and the concentration of the glabridin in the positive control solution was 125 µg/mL.

Test sample 1: the supramolecular 4-methoxysalicylic acid tranexamic acid ionic salt obtained in Example 1 was dissolved in a cell culture medium, and the concentrations of the supramolecular 4-methoxysalicylic acid tranexamic acid ionic salt in the test sample 1 were 0.078 mg/mL, 0.156 mg/mL, and 0.313 mg/mL, respectively.

Test sample 2: the tranexamic acid of Comparative Example 2 was dissolved in a cell culture medium, and the concentrations of the tranexamic acid in the test sample 2 were 0.078 mg/mL, 0.156 mg/mL, and 0.313 mg/mL, respectively.

Test sample 3: the potassium 4-methoxysalicylate of Comparative Example 1 was dissolved in a cell culture medium, and the concentrations of the potassium 4-methoxysalicylate in the test sample 3 were 0.078 mg/mL, 0.103 mg/mL, and 0.156 mg/mL, respectively.

### (2) Cell inoculation

Melanocytes were inoculated into a 6-well plate at a cell inoculation amount of 2.2×10⁵ melanocytes/well, and incubated overnight in an incubator (37 °C, 5% CO₂, 95% RH) for incubation overnight.

### (3) Administration

When the seeding efficiency of the cells in the 6-well plate reached 40% to 60%, administration was carried out in groups. The administration amount was 2 mL per well, and 3 replicate wells were set for each group. The cells were placed in an incubator (37 °C, 5% CO₂, 95% RH) for continuous culture for 72 h.

### (4) Sample collection

After the culture was completed, the remaining culture medium in the wells was aspirated and discarded, and 700 µL of 0.25% trypsin was added to each well to digest the melanocytes at 37 °C for 1 to 2 min; the cells were collected into a 1.5 mL centrifuge tube, centrifuged at 10,000 r/min for 10 min, and the supernatant was aspirated and discarded. 200 µL of distilled water, 500 µL of anhydrous ethanol, and 500 µL of diethyl ether were added to each tube and mixed uniformly; and the mixture was left at room temperature for 30 min and then centrifuged at 10,000 r/min for 10 min.

### (5) Dissolution

The supernatant was aspirated and discarded, and 1 mL of a 1 mol/L NaOH aqueous solution containing 10 wt% (volume fraction) DMSO was added to obtain the test samples of each group. The test samples of each group and the melanin standard solutions with different concentrations were respectively heated in a water bath at 80 °C for 30 min.

### (6) Reading

200 µL of the suspension was taken from each sample tube into a 96-well plate, 2 replicate wells were set, and the absorbance value was measured on an enzyme-linked immunosorbent detector at a wavelength of 405 nm.

According to the concentrations and absorbance values of the standard products, a melanin content-absorbance standard curve was drawn, the OD values of the samples were substituted into the standard curve, the melanin content was calculated, and statistical analysis was performed. The results are shown in Table 12.

**Table 12 Results of the inhibition rate of cellular melanin synthesis**

| Sample name | Concentration (mg/mL) | Cellular melanin content (%) | SD | P-value |
|---|---|---|---|---|
| Control | BC | 89.38 | 3.70 | / |
| | PC | 75.97 | 0.32 | <0.0001 |
| Test sample 1 | 0.078 | 79.48 | 2.47 | <0.0001 |
| | 0.156 | 76.53 | 2.47 | <0.0001 |
| | 0.313 | 63.47 | 1.21 | <0.0001 |
| Test sample 2 | 0.078 | 95.75 | 1.25 | Na |
| | 0.156 | 98.41 | 1.20 | Na |
| | 0.313 | 98.98 | 8.91 | Na |
| Test sample 3 | 0.078 | 80.76 | 1.35 | <0.01 |
| | 0.103 | 82.75 | 2.89 | <0.01 |
| | 0.156 | 79.86 | 1.98 | <0.01 |

As can be seen from Table 12, compared with the BC group, the melanin content of the PC group decreased significantly (P < 0.0001), indicating that this test is valid. Compared with the BC group, the melanin content of the supramolecular 4-methoxysalicylic acid tranexamic acid ionic salt of Example 1 decreased significantly and the effect is significantly better than that of potassium 4-methoxysalicylate and tranexamic acid, indicating that the supramolecular 4-methoxysalicylic acid tranexamic acid ionic salt has better whitening and spot-fading effects.

### Experimental Example 4 Transdermal effect test

The steps of the transdermal effect test are as follows.
(1) The back skin of GF Kunming mice was used, the subcutaneous fat layer and connective tissue were carefully peeled off, and the skin was rinsed clean with normal saline, and placed in normal saline for later use.
(2) The transdermal experiment was carried out by the Franz cell method, and the area of the mouse skin exposed in the diffusion cell of the Franz diffusion device was 1.13 cm², and the volume of the receiving chamber was 15 mL.
(3) 1.0 mL of the test drug solution was respectively taken and applied to the skin surface, 15 mL of a commercially available PBS buffered saline solution (pH ≈ 7.4) was added to the receiving cell as a receiving solution, and the device was placed in a constant-temperature water bath at 32 °C with a stirring speed of 350 rad/min, wherein the samples obtained in Experimental Examples 1 to 2 and Comparative Examples 1 to 3 were dissolved in an aqueous solution to obtain the test drug solutions with a concentration of 3 wt%.
(4) Test of the transdermal permeation amount: 2 mL of the receiving solution was respectively taken at different time points, 3 parallel portions were taken at each time point, and 2 mL of the receiving solution was supplemented into the receiving chamber immediately after sampling; and the receiving solutions taken at different time points were filtered through a 0.22 µm microporous filter membrane, and then the concentration of the receiving solution was measured by liquid phase detection.

The transdermal effect test results are shown in Table 13.

**Table 13 Transdermal effect test results**

| Group | Permeation amount per unit area (µg/cm²) | | | |
|---|---|---|---|---|
| | 2h | 5h | 21h | 24h |
| Experimental Example 1 | 12.34 | 123.46 | 1759.46 | 2230.47 |
| Experimental Example 2 | 11.39 | 115.63 | 1345.67 | 1852.64 |
| Comparative Example 1 | 6.57 | 84.32 | 500.79 | 841.36 |
| Comparative Example 2 | 10.96 | 113.29 | 1132.54 | 1354.74 |
| Comparative Example 3 | 9.87 | 110.95 | 1056.31 | 1285.67 |

As can be seen from Table 13, the transdermal effect of the supramolecular 4-methoxysalicylic acid tranexamic acid ionic salt of Example 1 is significantly better than that of the potassium 4-methoxysalicylate of Comparative Example 1, the tranexamic acid of Comparative Example 2, and the mixture of potassium 4-methoxysalicylate and tranexamic acid of Comparative Example 3, indicating that the supramolecular 4-methoxysalicylic acid tranexamic acid ionic salt has a better transdermal effect.

### Experimental Example 5 Patch test

The steps of the patch test are as follows.
(1) Qualified patch test equipment was selected, and by a closed patch test method, 0.020 g to 0.025 g of the test substance was placed in the patch test equipment, and externally applied and attached to the flexor side of the forearm of the subjects with hypoallergenic tape.

The supramolecular 4-methoxysalicylic acid tranexamic acid ionic salt of Example 1 was dissolved in deionized water to form a supramolecular 4-methoxysalicylic acid tranexamic acid ionic salt aqueous solution with a concentration of 3 wt%.

(2) The test substance was removed after 24 hours, and the skin reactions were observed at 0.5 h, 24 h, and 48 h after removal, respectively, and the results were recorded according to the skin reaction grading standards (as shown in Table 14) in the Safety and Technical Standards for Cosmetics (2015 edition).

**Table 14 Skin reaction grading standards for the skin closed patch test**

| Reaction degree | Score grade | Skin reaction |
|---|---|---|
| - | 0 | Negative reaction |
| ± | 1 | Doubtful reaction, with only slight erythema |
| + | 2 | Weakly positive reaction (erythema reaction): erythema, infiltration, edema, and possible papules |
| ++ | 3 | Strongly positive reaction (vesicle reaction): erythema, infiltration, edema, and possible papules and vesicles; the reaction extends beyond the test area |
| +++ | 4 | Extremely strong positive reaction (confluent vesicle reaction): obvious erythema, severe infiltration, edema, and confluent vesicle reaction; the reaction extends beyond the test area |

The statistical results of the patch test are shown in Table 15.

**Table 15 Patch test results**

| Group | Number of subjects | Observation time | Number of persons with different skin reaction grades in the patch test | | | | |
|---|---|---|---|---|---|---|---|
| | | | 0 | 1 | 2 | 3 | 4 |
| Test substance | 40 | 0.5h | 40 | 0 | 0 | 0 | 0 |
| | | 24h | 40 | 0 | 0 | 0 | 0 |
| | | 48h | 40 | 0 | 0 | 0 | 0 |
| Negative control | 40 | 0.5h | 40 | 0 | 0 | 0 | 0 |
| | | 24h | 40 | 0 | 0 | 0 | 0 |
| | | 48h | 40 | 0 | 0 | 0 | 0 |

As can be seen from Table 15, the supramolecular 4-methoxysalicylic acid tranexamic acid ionic salt of Example 1 was prepared into a 3 wt% aqueous solution for the human skin closed patch test, and 0 of the 40 subjects had adverse skin reactions; and this indicates that the supramolecular 4-methoxysalicylic acid tranexamic acid ionic salt has low irritation.

### Experimental Example 6 Test on whitening and spot-fading effects

### (1) Subject screening criteria

Volunteers aged between 18 and 60 years old who met the experimental requirements, with or without epidermal pigmentation in the skin of the forearm test area, were measured 5 times at different positions in the proposed test area with a tristimulus colorimeter, and the average ITA° value was between 10 and 41. The information of the subjects is shown in Table 16.

**Table 16 Information of the subjects**

| Number analvsis (persons) | | | | Age analysis/years old | | | Gender analysis | |
|---|---|---|---|---|---|---|---|---|
| Planned enrollment | Actual enrollment | Test completed | Included in analysis | Average age | Minimum age | Maximum age | Male | Female |
| 30 | 34 | 32 | 30 | 40.32 | 18 | 54 | 13 | 17 |

### (2) Test environment for the whitening and spot-fading effect

The whitening and spot-fading effect test was completed in a laboratory, the test temperature was 20 to 22 °C, and the relative humidity was 40% to 60%.

### (3) Requirements during the test

It is forbidden to use preparations that affect the whitening and spot-removing test on the tested part.

The subjects are forbidden to take preparations that affect the whitening and spot-removing test by drip, injection, oral administration, or other forms.

The subjects mainly engaged in indoor activities and avoided long-term exposure to light.

### (4) Preparation of the test sample

The supramolecular 4-methoxysalicylic acid tranexamic acid ionic salt of Experimental Example 1 was prepared into an essence according to the essence formula (as shown in Table 17).

**Table 17 Essence formula**

| No. | Ingredient name | Proportion (wt%) | No. | Ingredient name | Proportion (wt%) |
|---|---|---|---|---|---|
| 1 | Water | 87 | 8 | 1,3-propanediol | 3 |
| 2 | Glycerin | 4 | 9 | p-Hydroxyacetophenone | 0.5 |
| 3 | Glycereth | 2 | 10 | 1,2-Hexanediol | 0.5 |
| 4 | Betaine | 0.5 | 11 | 4-Methoxysalicylic acid tranexamic acid ionic salt | 2 |
| 5 | Sodium hyaluronate | 0.1 | | | |
| 6 | Disodium EDTA | 0.05 | 12 | Dipotassium glycyrrhizate | 0.05 |
| 7 | Xanthan gum | 0.25 | 13 | Arginine | 0.1 |

### (5) Application method of the test sample

Every morning and evening, 1.5 mL of the product was taken and evenly applied to the face.

### (6) Detection of skin data

Immediately after a single use of the test sample (i.e., day 0), and after 7 days, 14 days, 21 days, and 24 days, respectively, the skin color and facial data of the subjects before using the product were collected with a skin color measuring instrument (XJJC-EQ-029) and a facial scanner (XJJC-EQ-030), respectively.

### (7) Statistical method

Statistical analysis software was used for the statistical analysis of the data. The measurement data are expressed as: mean ± standard deviation, and a normal distribution test was performed to determine whether the normal distribution requirements are met; the comparison before and after self-treatment was performed by a paired t-test, otherwise by a two-related-samples rank-sum test; the comparison of grade data before and after use was performed by a two-related-samples rank-sum test; the comparison between the test area and the control area was performed by an independent-samples t-test or a rank-sum test; and at the same time, the regression coefficient (slope k value) of each parameter changing with time was calculated, and the significance levels were all P < 0.05.

The comparison results of the change rate of the facial brightness L value of the subjects are shown in FIG. 4. As can be seen from FIG. 4, after 7 days of using the essence of the supramolecular 4-methoxysalicylic acid tranexamic acid ionic salt, the facial brightness L value of the subjects increased, and the growth rate was higher than that of the blank essence group (the facial area to which no essence was applied), indicating that the essence of the supramolecular 4-methoxysalicylic acid tranexamic acid ionic salt can brighten the skin after 7 days of use.

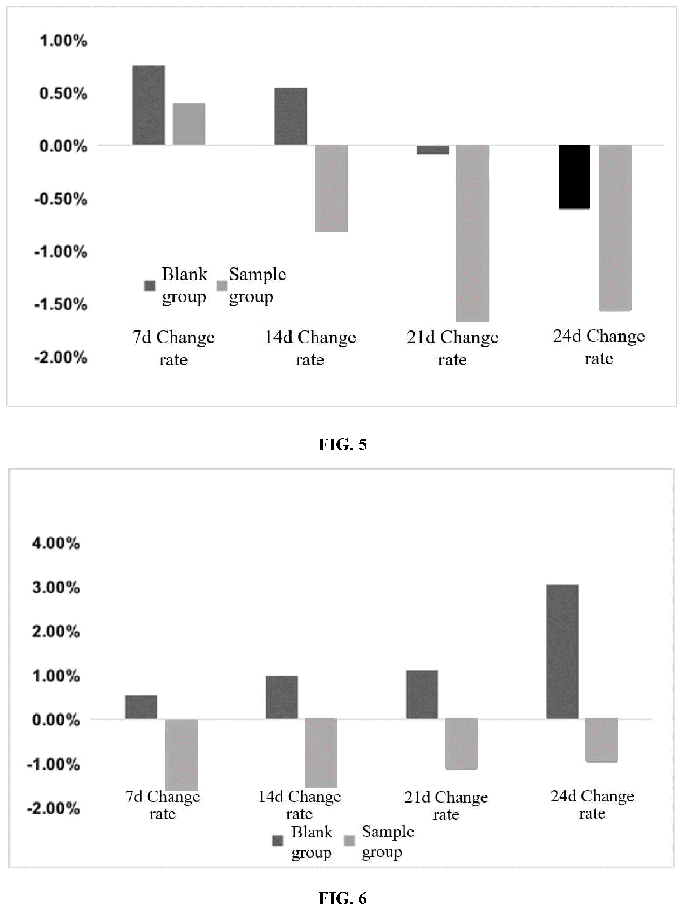

The comparison results of the change rate of the facial erythema E value of the subjects are shown in FIG. 5. As can be seen from FIG. 5, after 14 days of using the essence of the supramolecular 4-methoxysalicylic acid tranexamic acid ionic salt, the facial erythema E value of the subjects decreased, and the reduction rate was higher than that of the blank essence group (the facial area to which no essence was applied), indicating that the essence of the supramolecular 4-methoxysalicylic acid tranexamic acid ionic salt can achieve the effect of fading erythema after 14 days of use.

The comparison results of the change rate of the facial melanin M value of the subjects are shown in FIG. 6. As can be seen from FIG. 6, after 7 days of using the essence of the supramolecular 4-methoxysalicylic acid tranexamic acid ionic salt, the facial melanin M value of the subjects decreased, and the reduction rate was higher than that of the blank essence group (the facial area to which no essence was applied), indicating that the essence of the supramolecular 4-methoxysalicylic acid tranexamic acid ionic salt can achieve the effect of fading melanin after 7 days of use.

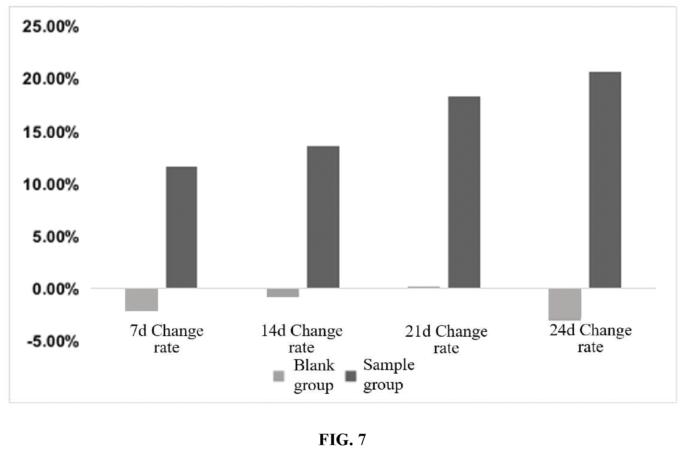

The comparison results of the change rate of the facial ITA° value of the subjects are shown in FIG. 7. As can be seen from FIG. 7, after 7 days of using the essence of the supramolecular 4-methoxysalicylic acid tranexamic acid ionic salt, the facial ITA° value of the subjects continued to increase, and the growth rate was much higher than that of the blank essence group (the facial area to which no essence was applied), indicating that the essence of the supramolecular 4-methoxysalicylic acid tranexamic acid ionic salt can whiten the skin after 7 days of use.

### Experimental Example 7 Clinical test for treating chloasma

The steps of the clinical test for treating chloasma are as follows.

### (1) Screening of subjects

First, a preliminary diagnosis of the chloasma condition was performed on the 40 recruited volunteers. The qualified subjects filled in informed consent forms. The subjects rested for 30 minutes in an environment with a temperature of 21 ± 1 °C and a relative humidity of 50 ± 10% RH.

Then, a VISIA skin image analyzer was used to scan the skin of the face, which uses the Mirror medical imaging system to perform group detection on skin of different age groups, cyclic facial photography technology and a small magnifying glass tool to quantitatively analyze redness, pores, and UV reflection points, and accurately diagnose subtle skin problems. A clear qualitative analysis report was provided.

### (2) Application of the test sample

According to the cream formula in Table 18, a cream was prepared by using the supramolecular 4-methoxysalicylic acid tranexamic acid ionic salt of Example 1 (recorded as the experimental group cream).

**Table 18 Cream formula**

| No. | Ingredient name | Proportion (wt%) | No. | Ingredient name | Proportion (wt%) |
|---|---|---|---|---|---|
| 1 | Cetearyl olivate | 1.8 | 13 | Stearyl alcohol | 1.4 |
| 2 | Sorbitan olivate | 1.2 | 14 | Allantoin | 0.15 |
| 3 | PEG-100 stearate | 1.5 | 15 | Cetyl alcohol | 1.6 |
| 4 | Glyceryl stearate | 1.5 | 16 | Acrylates/C10-30 alkyl acrylate crosspolymer | 0.15 |
| 5 | Caprylic/capric triglyceride | 6 | 17 | Xanthan gum | 0.2 |
| 6 | Ethylhexyl palmitate | 4 | 18 | Butylene glycol | 2 |
| 7 | Squalane | 2 | 19 | p-Hydroxyacetophenone | 0.5 |
| 8 | Polydimethylsiloxane | 2 | 20 | 1,2-Hexanediol | 0.5 |
| 9 | Water | 62.1 | 21 | 10 wt% sodium hydroxide aqueous solution | 1.8 |
| 10 | Glycerin | 4 | 22 | supramolecular 4-methoxysalicylic acid tranexamic acid ionic salt | 3 |
| 11 | Butylene glycol | 2 | 23 | Dipotassium glycyrrhizate | 0.1 |
| 12 | Trehalose | 0.5 | | | |

The cream was used for treatment; a commercially available anti-chloasma cream (recorded as the control group cream) was applied to one half of the face of the subjects, and the experimental group cream was applied to the other half of the face.

The cream was applied daily, and the subjects were followed up after 7 days, 14 days, and 28 days, respectively (the subjects could not wash their faces within 48 ± 4 hours before the visit), the facial skin condition was photographed, the facial condition was scored, and the subjects filled in questionnaires.

The requirements after applying the cream are as follows:

Do not squeeze or rub the skin of the facial part, otherwise it will cause damage to the facial skin surface and cause infection.

Avoid exposing the facial skin to excessively strong ultraviolet rays.

Avoid eating spicy and irritating food, smoking, drinking alcohol, etc.

The statistics of the questionnaire survey results for treating chloasma are shown in Table 19.

**Table 19 Statistical results of the questionnaire survey for treating chloasma**

| Group | Number of subjects | Observation time | Statistical table for evaluating the treatment effect on chloasma | | | | |
|---|---|---|---|---|---|---|---|
| | | | Chloasma area reduced | Chloasma color lightened | Irritation | Sticky and not fresh | No effect |
| Control group cream | 30 | 7 days | 0 | 4 | 3 | 2 | 21 |
| | | 14 days | 2 | 5 | 4 | 4 | 15 |
| | | 28 days | 4 | 6 | 6 | 4 | 10 |
| Experimental group cream | 30 | 7 days | 1 | 8 | 0 | 0 | 21 |
| | | 14 days | 5 | 15 | 0 | 0 | 10 |
| | | 28 days | 10 | 15 | 0 | 0 | 5 |

As can be seen from Table 19, the cream prepared by using the supramolecular 4-methoxysalicylic acid tranexamic acid ionic salt of Example 1 (i.e., the experimental group cream) has the effect of treating chloasma, and is significantly better than a certain commercially available competing anti-chloasma cream (i.e., the control group cream); and this indicates that the supramolecular 4-methoxysalicylic acid tranexamic acid ionic salt has a good anti-chloasma effect.

In summary, the supramolecular 4-methoxysalicylic acid tranexamic acid ionic salt provided by the present invention can not only effectively solve the problem of poor transdermal permeability of potassium 4-methoxysalicylate, but also effectively reduce the toxicity and side effects and irritation of potassium 4-methoxysalicylate and tranexamic acid when used alone, thereby expanding the application range thereof to a great extent. In addition, the 4-methoxysalicylic acid structure and the tranexamic acid structure in the supramolecular 4-methoxysalicylic acid tranexamic acid ionic salt have a synergistic effect, and compared with potassium 4-methoxysalicylate used alone or tranexamic acid used alone, the supramolecular 4-methoxysalicylic acid tranexamic acid ionic salt can effectively enhance the whitening effect, brightening effect, spot-fading effect, anti-yellowing effect, acne mark fading effect, and transdermal effect. Therefore, the supramolecular 4-methoxysalicylic acid tranexamic acid ionic salt can be used in the preparation of drugs, cosmetics, and skincare products having the effects of whitening, spot fading, acne mark fading, etc.

The embodiments described above are part of the embodiments of the present invention, rather than all of the embodiments. The detailed description of the embodiments of the present invention is not intended to limit the scope of the claimed present invention, but merely represents the selected embodiments of the present invention. Based on the embodiments of the present invention, all other embodiments obtained by those of ordinary skill in the art without creative work shall fall within the protection scope of the present invention.

In addition, it should be noted that the specific technical features described in the above detailed description can be combined in any suitable manner as long as they are not contradictory, and in order to avoid unnecessary repetition, the various possible combinations will not be described separately in the present invention.

### INDUSTRIAL APPLICABILITY

In summary, the supramolecular 4-methoxysalicylic acid tranexamic acid ionic salt provided by the present invention can not only effectively solve the problem of poor transdermal permeability of potassium 4-methoxysalicylate, but also effectively reduce the toxicity and side effects and irritation of potassium 4-methoxysalicylate and tranexamic acid when used alone, thereby expanding the application range thereof to a great extent. In addition, the 4-methoxysalicylic acid structure and the tranexamic acid structure in the supramolecular 4-methoxysalicylic acid tranexamic acid ionic salt have a synergistic effect, and compared with potassium 4-methoxysalicylate used alone or tranexamic acid used alone, the supramolecular 4-methoxysalicylic acid tranexamic acid ionic salt can effectively enhance the whitening effect, brightening effect, spot-fading effect, anti-yellowing effect, acne mark fading effect, and transdermal effect. Therefore, the supramolecular 4-methoxysalicylic acid tranexamic acid ionic salt can be used in the preparation of drugs, cosmetics, and skincare products having the effects of whitening, spot fading, acne mark fading, etc.

## Claims

1. A supramolecular 4-methoxysalicylic acid tranexamic acid ionic salt, **characterized in that** the supramolecular 4-methoxysalicylic acid tranexamic acid ionic salt is a compound represented by a following structural formula, or the supramolecular 4-methoxysalicylic acid tranexamic acid ionic salt is a hydrate of the compound represented by the following structural formula:

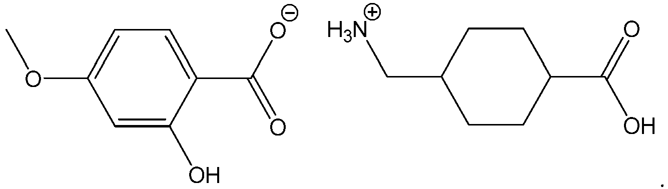

2. The supramolecular 4-methoxysalicylic acid tranexamic acid ionic salt according to claim 1, wherein the supramolecular 4-methoxysalicylic acid tranexamic acid ionic salt is of an orthorhombic crystal system.

3. The supramolecular 4-methoxysalicylic acid tranexamic acid ionic salt according to any one of claims 1 to 2, wherein a chemical formula of Formula I is C₁₆H₂₃NO₆, and a chemical formula of the hydrate of Formula I is C₁₆H₂₃NO₆·nH₂O, where n is a natural number.

4. The supramolecular 4-methoxysalicylic acid tranexamic acid ionic salt according to any one of claims 1 to 3, wherein the supramolecular 4-methoxysalicylic acid tranexamic acid ionic salt has a P2₁2₁2₁ space group, with unit cell parameters of a = 7.9541(6) Å, b = 12.9509(12) Å, c = 16.5051(13) Å, α = 90°, β = 90°, γ = 90°, the number of units Z in the unit cell = 4, and a unit cell volume of 1700.2(2) Å³.

5. A method for preparing the supramolecular 4-methoxysalicylic acid tranexamic acid ionic salt according to any one of claims 1 to 4, **characterized in that** the method comprises:
subjecting a liquid phase containing 4-methoxysalicylic acid, tranexamic acid, and an organic solvent to a heating and stirring treatment and an ultrasonic treatment in sequence, to obtain a supramolecular 4-methoxysalicylic acid tranexamic acid ionic salt solution; and
subjecting the supramolecular 4-methoxysalicylic acid tranexamic acid ionic salt solution to a crystallization treatment.

6. The method according to claim 5, wherein a molar ratio of the 4-methoxysalicylic acid to the tranexamic acid is (3 to 1): (1 to 3).

7. The method according to any one of claims 5 to 6, wherein a temperature of the heating and stirring treatment is 80 °C to 110 °C; and/or
a duration of the heating and stirring treatment is 10 h to 25 h; and/or
the heating and stirring treatment adopts oil bath heating.

8. The method according to any one of claims 5 to 7, wherein a temperature of the ultrasonic treatment is 40 °C to 60 °C; and/or
an ultrasonic frequency of the ultrasonic treatment is 20 kHz to 60 kHz; and/or
an ultrasonic power of the ultrasonic treatment is 280 W to 320 W; and/or
a duration of the ultrasonic treatment is 4 h to 8 h.

9. The method according to any one of claims 5 to 8, wherein a temperature of the crystallization treatment is ≤ 0 °C; and/or
the crystallization treatment is carried out under a vacuum condition.

10. The method according to any one of claims 5 to 9, wherein a preparation method for the supramolecular 4-methoxysalicylic acid tranexamic acid ionic salt solution further comprises: after the ultrasonic treatment, subjecting a system after the ultrasonic treatment to a concentration treatment; and/or
the method for preparing the supramolecular 4-methoxysalicylic acid tranexamic acid ionic salt further comprises: after the crystallization treatment, sequentially filtering, washing, and drying a system after the crystallization treatment.

11. The method according to any one of claims 5 to 10, wherein the organic solvent comprises at least one of methanol, ethanol, isopropanol, N-methylpyrrolidone, tetrahydrofuran, benzene, toluene, petroleum ether, n-hexane, acetone, dioxane, N,N-dimethylformamide, ethyl acetate, and acetonitrile; and/or
a preparation process of the supramolecular 4-methoxysalicylic acid tranexamic acid ionic salt is carried out under an inert gas atmosphere;
optionally, the inert gas comprises at least one of nitrogen, helium, neon, and argon.

12. The method according to any one of claims 5 to 11, wherein a preparation method for the liquid phase containing the 4-methoxysalicylic acid, the tranexamic acid, and the organic solvent comprises: dispersing the 4-methoxysalicylic acid and the tranexamic acid in the organic solvent respectively to obtain a 4-methoxysalicylic acid dispersion and a tranexamic acid dispersion, and then adding the 4-methoxysalicylic acid dispersion dropwise to the tranexamic acid dispersion, to obtain a mixed solution as the liquid phase.

13. The method according to any one of claims 5 to 12, wherein during a preparation process of the supramolecular 4-methoxysalicylic acid tranexamic acid ionic salt, an inert gas is supplemented to maintain a constant temperature and a constant pressure.

14. Use of the supramolecular 4-methoxysalicylic acid tranexamic acid ionic salt according to any one of claims 1 to 4 in the preparation of a drug, a cosmetic, or a skincare product.

15. The use according to claim 14, wherein a mass percentage of the supramolecular 4-methoxysalicylic acid tranexamic acid ionic salt in the drug, the cosmetic, or the skincare product is 1-5%.
